# EUROPEAN PATENT SPECIFICATION

(11) **EP 3 785 679 B1**
(45) Date of publication and mention of the grant of the patent: **02.10.2024**
(21) Application number: 20192141.8
(22) Date of filing: 21.08.2020
(51) Int. Cl.: A61F 5/00, A61B 17/11

(54) **A DEGRADABLE INTESTINAL DIVERSION DEVICE**
ABBAUBARE INTESTINALE ABLENKVORRICHTUNG
DISPOSITIF DE DÉRIVATION INTESTINALE DÉGRADABLE

(30) Priority: 27.08.2019 CN 201910794214
(43) Date of publication of application: 03.03.2021
(73) Proprietor: Zhejiang University, Hangzhou 310027 (CN)
(72) Inventor: Cai, Xiujun, Hangzhou, 310016 (CN); Wu, Zhongyu, Hangzhou, 310016 (CN); Chen, Mingyu, Hangzhou, 310016 (CN); Huang, Diyu, Hangzhou, 310016 (CN); Wang, Yifan, Hangzhou, 310016 (CN); Shi, Lei, Hangzhou, 310016 (CN); Dai, Weijian, Hangzhou, 310016 (CN); Ma, Yanli, Hangzhou, 310016 (CN)
(74) Representative: karo IP

(56) References cited:
- CN-A- 105 078 535
- CN-U- 204 500 833
- CN-Y- 201 216 623
- US-A1- 2018 078 745

## Description

### Technical field

The invention relates to a degradable intestinal diversion device, which is used to assist a drainage tube to complete the intestinal diversion.

### Background

After colorectal surgery, anastomotic leakage is a common complication, and its incidence is about 2%-20%. After the intestinal lesion is removed, the stump of the intestine needs to be anastomosed to restore its continuity. If the intestinal anastomosis is not well healed, anastomotic leakage occurs, and the contents of the intestine flow into the abdominal cavity, which may cause abdominal or even systemic infection, and cause septic shock and even death when the situation is serious. According to reports in the literature, the incidence of anastomotic leakage after colorectal surgery is approximately 2%-20%. The lower the anastomotic position (closer to the anus), the higher the incidence of anastomotic leakage. Anastomotic leakage not only increases the incidence of other complications and mortality of patients, and increases the suffering of patients, but also increases the medical burden. Patients with anastomotic leakage are more likely to endure intestinal stenosis, difficulty defecation, urgency, incontinence, and worse tumor prognosis, and they are likely to undergo secondary surgery, permanent stoma and other treatments. Therefore, after various types of colorectal surgery, especially after low rectal cancer surgery, in order to avoid anastomotic leakage and its complications, most patients need intestinal protective ostomy. The most commonly used is terminal ileal protective ostomy surgery. Traditional ileal intubation protective ostomy surgery only inserts a fistula tube into the ileum, which cannot guarantee complete blockage of the intestinal lumen, and the fecal diversion is incomplete, and the protective effect of the anastomosis is not reliable, so its application is limited.

The intestinal protective stoma is a commonly used protective operation after colorectal surgery which can be applied in any situation where the distal anastomosis needs to be protected or the distal intestine needs to be left open. Usually the terminal ileum is raised and then a loop or double-chamber stoma is made, and the distal anastomosis is left open to completely divert the feces to avoid the impact and pollution of the feces on the anastomosis, creating favorable conditions for the anastomosis healing, and promoting the anastomotic healing .

Intestinal protective stoma can reduce the complications and related mortality caused by anastomotic leakage. However, patients who have received a protective stoma need to undergo a second operation 1 to 6 months after surgery to close the temporary stoma, and restore intestinal continuity, and return the stoma to the abdominal cavity. When a patient lives with a stoma, he not only has to endure the inconvenience caused by stoma care, but also pay a higher economic cost. The diversion material of the ileostomy has not yet formed a shaped stool, and contains a large amount of digestive juice that is much corrosive to the skin around the stoma. Improper care will cause greater pain to the patient and seriously affect the quality of life .

Therefore, in clinical diagnosis and treatment, there is an urgent need for a new type of intestinal diversion device specially designed for patients who need protective intestinal diversion, which can completely divert feces from the artificial stoma and avoid the second surgery of the stoma, while reducing skin irritation around the stoma. The equipment needs to be convenient to use, easy to care for, safe, effective, economical and practical, which not only reduces the suffering of patients, but also saves medical expenses.

Chinese patent CN201510840170 discloses a functional intestinal ostomy sleeve, which is composed of a drainage tube for guiding feces to the outside of the body, several sacs for blocking intestinal fluid, and several inflation tubes for inflating the sacs. The use of this sleeve for protective ostomy avoids secondary operation and can completely divert feces. However, after the operation, the air sacs containing air remain in the body. If they are damaged, the escaped air may cause intestinal infections and endanger intestinal health. In addition, this method of blocking the intestinal lumen by inflating sacs to compress the intestine is difficult to grasp in actual use and has a high risk. If the inflation pressure is not enough, the intestinal lumen cannot be completely blocked and the flow cannot be completely diverted; If the inflation pressure is too high, it may over-press the intestine, causing necrosis of the intestinal wall in severe cases, and even causing serious consequences such as perforation and intestinal leakage.

Chinese patent application CN 105078535 A discloses a degradable intestinal drainage stent. US2018078745 discloses a drainage stent which maintains an open flow passage between fused tissue layers.

### Summary of the invention

In order to overcome the shortcomings of the existing protective intestinal stoma that requires a second operation to reenter the stoma, and the traditional ileal intubation stoma cannot completely divert feces, the present invention provides a degradable intestinal diversion device that can be used to completely divert feces and automatically close the fistula and require no second operation.

In order to solve the above technical problems, the present invention designs a degradable intestinal diversion device used with common drainage tubes, such as T-shaped drainage tubes or mushroom drainage tubes.

The degradable intestinal diversion device of the present invention includes an intestinal fixation part, wherein the intestinal fixation part includes a circular tube constituting a bridge for intestinal incisions on both sides to crawl towards each other and anastomosed; the two ends of the circular tube are respectively connected to a gradually expanding flared opening, and a convex ring for binding a broken end of the intestine is arranged on the circular tube near each flared opening, and a hollow inner passage of the circular tube is provided with a barrier film for blocking the hollow inner passage; the intestinal fixation part and the barrier film are made of biocompatible degradable materials.

Preferably, the barrier film and the flared opening are integrally formed with the circular tube.

Preferably, the biocompatible degradable material uses polyglycolic acid (PGA).

Preferably, the biocompatible degradable material is also doped with barium sulfate.

The barrier film can completely prevent the feces from flowing into the downstream intestines, so that the feces can be completely diverted out of the body through the drainage tube, thereby fully protecting the downstream anastomosis. After passing the dangerous period of anastomotic leakage, the device begins to degrade; when the anastomosis is almost completely anastomosed, the device completely disintegrate; after that, it is completely degraded into water and carbon dioxide. Therefore, the intestinal protective ostomy with the use of the device and the intubation can completely block the feces and allow it to be discharged from the body through the drainage tube, without the need for a second operation of stoma reinjection, and no infection or damage to the wall of intestines.

The present invention uses polyglycolic acid with good biocompatibility and degradability. Under an in vitro environment with a temperature of 37±1°C and a pH of 7.4±0.2, the device keeps intact for 10 days, and degrades and completely disintegrates after 28 days. The clinically observed intestinal anastomotic leakage usually occurs within 7 days after the operation, and the anastomosis is usually completely anastomosed about 30 days after the operation. Therefore, the device of the invention can protect the anastomosis during the dangerous period of intestinal anastomotic leakage and avoid the second surgery to remove the device.

The beneficial effects of the present invention are: 1. The device is simple and convenient to insert into the intestinal cavity, and it is fixed with a purse string, which is obviously faster than the traditional loop stoma or double cavity stoma; 2. The device with a barrier film can completely block the intestine to divert the contents of the intestine out of the body from the diversion drainage tube, which can temporarily protect the distal anastomosis. After the distal anastomosis is healed, the diversion tube should be removed to avoid secondary operations; 3. During use It does not need to rely on the operator's experience and is easy to master. It not only ensures complete blockage of the intestinal cavity, but also avoids compression of the intestine; 4. Using the device and the drainage tube overcomes the infection or necrosis of the intestinal wall that may be caused by the breakage of the sacs or the compression to the intestine ; 5. The material of the device also contains a barium sulfate imaging agent, which can be visualized in X-ray or CT examination to facilitate the dynamic observation of the degradation of the device; 6. The fistula tube is connected to the external abdominal wall fistula bag without negative pressure, and patients can eat non-slag semi-flow food, with good diverting effect and little irritation to the skin at the stoma; 7. Mushroom tubes can be used as diverting drainage tubes according to different needs, and the pipeline can be flushed.

### Description of the drawings

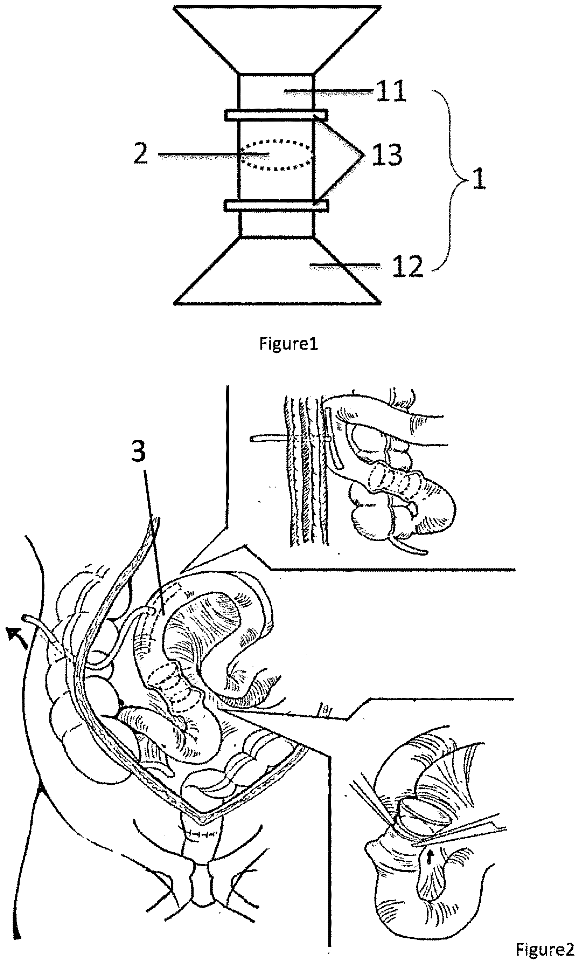

Figure 1 is a front view of the present invention.
Figure 2 is a schematic diagram of the present invention used with a drainage tube.

### Embodiments

The technical scheme of the present invention will be further explained below in conjunction with the drawings.

The degradable intestinal diversion device of the present invention made of biocompatible degradable materials and used with the drainage tube 3 is composed of an intestinal fixing part 1 and a barrier film 2.

The human body biocompatible and degradable material used in the present invention for manufacturing the device, such as polyglycolic acid (PGA), is mixed with an appropriate amount of barium sulfate.

The intestinal fixation part 1 includes a circular tube 11 constituting the bridge for the intestinal incisions on both sides to crawl towards each other and anastomosed ; the two ends of the circular tube 11 are respectively connected with a a gradually expanding flared opening 12, and a convex ring 13 for binding the broken end of the intestine is arranged on the circular tube 11 near each flared opening, and the hollow inner passage of the circular tube 11 is provided with a barrier film 2 that blocks the hollow inner passage.

Preferably, the barrier film 2 and the flared opening 12 are integrally formed with the circular tube 11.

The barrier film 2 and the flared opening 12 are integrally formed with the circular tube 11.

The method of using the above-mentioned device for diversion operation is that after cutting off the terminal ileum intestine, measure the diameter of the intestine, and then select a degradable intestinal diversion device of appropriate specifications according to the diameter of the intestine. When diverting, the device is placed in the intestinal cavity, and the ileum is fixed on the device with a thread at the convex ring 13. The two broken ends of the ileum are in contact at the midpoint of the longitudinal axis of the device. The incision edges of the two ends should be completely aligned. Sutures can be used for full-thickness intermittent suture. After that, a small opening is opened in the proximal small intestine about 15 cm away from the device, and a T-shaped drainage tube or mushroom drainage tube is implanted. The drainage tube 3 penetrates the body surface and is fixed, and the drainage bag is connected.

The degradable device for the degradable intestinal device of the present invention is made of biocompatible polyglycolic acid (PGA) material and contains barium sulfate. The length of the device is about 3-5cm, and the diameter range is 15-20mm. It is suitable for different patient's intestinal size. There is a degradable barrier film inside the device, the material of which is the same as that of the device. After the device is placed in the small intestine, the intestinal lumen can be completely blocked. The device contains barium sulfate imaging agent, which can be visualized in X-ray or CT inspection, which is convenient for dynamic observation of device degradation.

The degradable complete intestinal diversion device is made of polyglycolic acid (PGA) as the main material, mixed with 12.75% by weight of barium sulfate as the imaging agent. In vitro experiment it keeps intact for 10 days, and it degrades and completely disintegrates after 28 days. The sample used in this embodiment is a circular tube with flared opening at each of the both ends, with a total length of about 20 mm, an inner diameter of 18 mm, an outer diameter of 23 mm, a flared opening length of 5 mm, a wall thickness of 1 mm, and a barrier film thickness of about 0.75 mm. The design with a flared opening at each of the both ends of the device is convenient for the device to support the intestine and fix the device. The barrier film inside the device can completely block the contents of the intestine and accomplish complete diversion.

Place the device into the end of the ileum and fix it with an absorbable purse string. Insert the stoma drainage tube (such as mushroom drainage tube) in the proximal ileum where the device is placed against the direction of intestinal peristalsis (that is, the upstream intestine of the small intestine where the device is placed ). One end of the drainage tube is pulled out of the abdominal wall and fixed on the abdominal skin. The intubation and the intestinal wall of the small intestine is fixed with an absorbable thread around the tube. Since the barrier film in the degradable device blocks the hollow inner passage of the device, the intestinal contents cannot enter the distal intestinal segment through the device, thereby avoiding the impact and contamination of the intestinal contents on the distal anastomosis. The degradable device can disintegrate in about 30 days and be discharged with the contents of the intestine. Clinically observed intestinal anastomotic leakage usually occurs within 7 days after the operation, and the anastomosis is usually completely healed about 30 days after the operation. After it is observed that the device has collapsed and left the original position by taking a plain radiograph of the abdomen and an angiography through the drainage tube, the drainage tube can be clamped. If there is no obvious intestinal obstruction, anastomotic leakage and other complications, the drainage tube can be removed and the abdominal wall incision can be sutured. A second operation is avoided.

The features of the present invention are:
1. It is a degradable and biocompatible intestinal diversion device. The final degradation products are carbon dioxide and water.
2. The device is inserted into the small intestine by the way of internal support and suture-free, which is convenient and quick to operate.
3. The device contains a developer and can be developed under X-ray. The constituent materials of each part of the device are the same (a mixture of polyglycolic acid and barium sulfate).
4. The degradation time of the device in the human body is about 20-30 days. The diversion device can disintegrate by itself and be excreted with feces, without the need for a second operation to remove the device.
5. The device contains a barrier film that can completely block the intestinal cavity. The diversion device can completely block the intestinal contents, so that the intestinal contents can only be drawn out of the body through the diversion drainage tube of the upstream intestine, and play the role of completely diverting the intestinal contents.
6. The validity period (protection period) of the diversion device is about 20-28 days. After the expiration of the diversion period, the intestinal patency is automatically restored, without the need for a second operation to return to the stoma.

This embodiment is only to illustrate the technical solution of the present invention, not to limit the protection scope of the present invention.

## Claims

1. A degradable intestinal diversion device, comprising an intestinal fixation part (1), wherein the intestinal fixation part (1) includes a circular tube (11) constituting a bridge for intestinal incisions on both sides to crawl towards each other and anastomose; the two ends of the circular tube (11) are respectively connected to a gradually expanding flared opening (12), and a convex ring (13) for binding a broken end of the intestine is arranged on the circular tube (11) near each flared opening (12), wherein the circular tube defines a hollow inner passage **characterized in that** the hollow inner passage is provided with a barrier film (2) for blocking the hollow inner passage; the intestinal fixation part (1) and the barrier film (2) are made of biocompatible degradable materials.

2. The degradable intestinal diversion device according to claim 1, wherein the barrier film (2) and the flared opening (12) are integrally formed with the circular tube (11).

3. The degradable intestinal diversion device according to claim 1, wherein the biocompatible degradable material is polyglycolic acid.

4. The degradable intestinal diversion device according to claim 1, wherein the biocompatible degradable material is doped with barium sulfate.

## Patentansprüche

1. Eine abbaubare Darmableitungsvorrichtung, die einen Darmfixierungsteil (1) umfasst, wobei der Darmfixierungsteil (1) ein Rundrohr (11) einschließt, das eine Brücke für beidseitige Darmschnitte bildet, die aufeinander zu laufen und zusammenmünden; die beiden Enden des Rundrohrs (11) sind jeweils mit einer sich allmählich erweiternden Öffnung (12) verbunden und ein konvexer Ring (13) zum Befestigen eines unterbrochenen Darmendes ist auf dem Rundrohr (11) in der Nähe jeder der sich erweiternden Öffnungen (12) angeordnet, wobei das Rundrohr einen hohlen inneren Durchgang definiert, **dadurch gekennzeichnet, dass** der hohle innere Durchgang mit einem Barrierefilm (2) zum Blockieren des hohlen inneren Durchgangs versehen ist; das Darmfixierungsteil (1) und der Barrierefilm (2) sind aus biokompatiblen abbaubaren Materialien hergestellt.

2. Abbaubare Darmableitungsvorrichtung nach Anspruch 1, wobei der Barrierefilm (2) und die sich erweiternde Öffnung (12) in einem Stück mit dem Rundrohr (11) ausgebildet sind.

3. Abbaubare Darmableitungsvorrichtung nach Anspruch 1, wobei das biokompatible abbaubare Material Polyglykolsäure ist.

4. Abbaubare Darmableitungsvorrichtung nach Anspruch 1, wobei das biokompatible abbaubare Material mit Bariumsulfat dotiert ist.

## Revendications

1. Dispositif de dérivation intestinale dégradable, comprenant une partie de fixation intestinale (1), dans lequel la partie de fixation intestinale (1) comprend un tube circulaire (11) constituant un pont pour les incisions intestinales des deux côtés pour ramper l'une vers l'autre et s'anastomoser ; les deux extrémités du tube circulaire (11) sont respectivement reliées à une ouverture évasée à expansion progressive (12), et un anneau convexe (13) pour lier une extrémité cassée de l'intestin est disposé sur le tube circulaire (11) près de chaque ouverture évasée (12), dans lequel le tube circulaire définit un passage interne creux, **caractérisé en ce que** le passage interne creux est pourvu d'un film barrière (2) pour bloquer le passage interne creux ; la partie de fixation intestinale (1) et le film barrière (2) sont constitués de matériaux dégradables biocompatibles.

2. Dispositif de dérivation intestinale dégradable selon la revendication 1, dans lequel le film barrière (2) et l'ouverture évasée (12) sont formés d'un seul tenant avec le tube circulaire (11).

3. Dispositif de dérivation intestinale dégradable selon la revendication 1, dans lequel le matériau dégradable biocompatible est l'acide polyglycolique.

4. Dispositif de dérivation intestinale dégradable selon la revendication 1, dans lequel le matériau dégradable biocompatible est dopé au sulfate de baryum.
